# EUROPEAN PATENT APPLICATION

(11) **EP 4 512 391 A1**
(43) Date of publication of application: **26.02.2025**
(21) Application number: 23792040.0
(22) Date of filing: 23.03.2023
(51) Int. Cl.: A61K 9/107, A61K 47/36

(54) **DRUG DELIVERY SYSTEM AND MANUFACTURING METHOD THEREFOR**

(30) Priority: 21.04.2022 KR 20220049433
(71) Applicant: Mepion Co., Ltd., Seoul 08505 (KR)
(72) Inventor: NAM, Jung Hyuk, Seongnam-si Gyeonggi-do 13623 (KR); CHOI, Doo Yeol, Dangjin-si Chungcheongnam-do 31743 (KR); KANG, Min Kyu, Seoul 04945 (KR); PARK, Sun Hee, Seoul 08745 (KR)
(74) Representative: dompatent von Kreisler Selting Werner - Partnerschaft von Patent- und Rechtsanwälten mbB
(86) International application number: PCT/KR2023/003844
(87) International publication number: WO 2023/204460

(57) **Abstract**

Disclosed are a drug delivery system and a method for producing the same. A method for producing a drug delivery system according to one embodiment of the present invention may include: a micelle preparation step of preparing polymer micelles by mixing and stirring a biodegradable polymer solution and an oil; a bead preparation step of preparing hydrogel beads filled with the oil therein by crosslinking the polymer micelles; a hollow bead preparation step of preparing hollow beads by removing the oil contained in the hydrogel beads; and a filling step of filling the hollow beads with an additive.

## Description

### Technical Field

The present disclosure relates to a drug delivery system and a method for producing the same.

### Background Art

In general, a drug delivery system is a carrier that is injected *in vivo* and releases or delivers a substance having pharmacological activity. In connection with this, Korean Patent No. 10-2278255 discloses a technology relating to a drug delivery system.

Hyaluronic acid, one of the materials that make up drug delivery systems, is a biopolymer that is present in the body and is widely used for medical or cosmetic purposes due to its excellent biocompatibility. However, there is a problem in that since hyaluronic acid itself is degraded in the human body within several hours, it is difficult to control the rate of drug release from the drug delivery system to a slow rate.

Conventionally, studies have been conducted to increase the *in vivo* persistence of hyaluronic acid through crosslinking. However, when the content of the crosslinking agent is increased during production in order to increase the *in vivo* persistence time of hyaluronic acid, there is a concern that the crosslinking agent may be recognized as a foreign substance in the human body, causing an inflammatory response. In contrast, when the content of the crosslinking agent is decreased, a problem arises in that the obtained material has low viscoelasticity and is degraded in the human body within a short period of time. Therefore, there is a need for the development of a sustained-release drug delivery system that can control the drug release rate to a slow rate while reducing the content of the crosslinking agent.

Meanwhile, in the existing production method of making beads through a mechanical processing process (e.g., grinding) after preparing a crosslinked hydrogel, it is not easy to control the diameter of the bead during grinding, and the surface of the bead is uneven or the bead has a distorted shape, indicating that it is not easy to make beads having a round shape and a smooth surface like balls.

In addition, in the case of homogeneously crosslinked hydrogel beads in which the inside and outside of the beads are crosslinked with a uniform degree of crosslinking, there are problems in that it is difficult to load a useful substance because the space inside the bead is narrow, and in that it is difficult to apply a sustained-release technology that releases a useful substance in a constant and sustained manner.

### Disclosure

### Technical Problem

An object of the technical spirit of the present disclosure is to solve the above-described problems, and an object of the present disclosure is to provide a technology capable of preparing hydrogel beads which may be loaded with useful substances therein and may release the useful substances over time.

Another object of the technical spirit of the present disclosure is to provide a technology capable of appropriately controlling the content of a crosslinking agent during the preparation of hydrogel beads.

Still another object of the technical spirit of the present disclosure is to provide a technology capable of preparing hydrogel beads having a spherical shape.

The objects to be achieved by the present invention are not limited to the objects described above, and other objects not mentioned above will be clearly understood by those skilled in the art to which the present invention pertains from the following description.

### Technical Solution

To achieve the above objects, as an aspect of the present invention, a method for producing a drug delivery system according to one embodiment of the present invention may include: a micelle preparation step of preparing polymer micelles by mixing and stirring a biodegradable polymer solution and an oil; a bead preparation step of preparing hydrogel beads filled with the oil therein by crosslinking the polymer micelles; a hollow bead preparation step of preparing hollow beads by removing the oil contained in the hydrogel beads; and a filling step of filling the hollow beads with an additive.

In addition, a crosslinking agent that is used in the bead preparation step may be at least one selected from the group consisting of 1-ethyl-3-(3-dimethylaminopropyl)carbodiimide hydrochloride, N,N'-dicyclohexylcarbodiimide, butanediol diglycidyl ether, polyethylene glycol diglycidyl ether, N-hydroxysuccinimide, N-hydroxysulfosuccinimide, and combinations thereof.

In addition, the biodegradable polymer contained in the biodegradable polymer solution may be at least one selected from among hyaluronic acid and a hyaluronic acid salt.

In addition, the additive may be at least one selected from among a drug, a protein, and a genetic material.

In addition, the drug may be an anticancer drug.

In addition, the genetic material may be at least one selected from among messenger RNA (mRNA) and plasmid DNA (pDNA).

Meanwhile, the method for producing a drug delivery system may further include: a mixing step of preparing a mixture by mixing the hollow beads with a solvent; a surface modification step of adding a cationic crosslinker and an activator to the mixture; a stirring step of stirring the mixture; a filtering step of filtering the mixture to remove residual material; and a drying step for freeze-drying the filtered beads.

In addition, the cationic crosslinker may be at least one selected from among lysine, tris(2-ethylhexyl)amine, bis(2-ethylhexyl)amine, tris(2-pyridylmethyl)amine, poly(ethylene glycol)methyl ether amine, tris(2-aminoethyl)amine, hydrazine, poly(ethylene glycol)bis(amine), tris[2-(isopropylamino)ethyl]amine, and ethylenediamine.

In addition, the activator may be at least one selected from among 1-ethyl-3-(3-dimethylaminopropyl)carbodiimide hydrochloride, N,N'-dicyclohexylcarbodiimide, N,N'-carbonyldiimidazole, and N-hydroxysuccinimide.

In order to achieve the above objects, as another aspect of the present invention, a drug delivery system according to another embodiment of the present invention may be produced by the above-described method for producing a drug delivery system.

The above-described technical solutions are merely exemplary and should not be construed as limiting the present invention. In addition to the above-described exemplary embodiments, there may be additional embodiments disclosed in the drawings and the detailed description of the invention.

### Advantageous Effects

As described above, according to various embodiments of the present invention, it is possible to achieve a sustained-release function of sustainedly releasing a functional additive from beads over time by making biodegradable hollow beads and filling the inside of the biodegradable hollow beads with the functional additive.

In addition, according to various embodiments of the present invention, it is possible to produce a large amount of hydrogel beads having a round surface and a smooth spherical shape like balls.

In addition, according to various embodiments of the present invention, it is possible to produce hydrogel beads by appropriately controlling the content of the crosslinking agent.

The effects according to various embodiments of the present invention are not limited to the effects mentioned above, and other effects not mentioned above will be clearly understood by those skilled in the art from the description of the claims.

### Description of Drawings

FIG. 1 is a flow chart illustrating a method for producing a drug delivery system according to one embodiment of the present invention;
FIG. 2 is a perspective view schematically illustrating the structure of an impeller that is used in a micelle preparation step according to one embodiment of the present invention;
FIG. 3 is a conceptual diagram schematically illustrating the structure of a ball mill device that is used in a micelle preparation step according to another embodiment of the present invention;
FIG. 4 is a confocal micrograph of hydrogel beads according to Example 1 of the present invention, taken at ×100 magnification;
FIG. 5 is a graph showing the results of analyzing hydrogel beads according to Example 1 of the present invention using a nano-particle size analyzer, and
FIG. 6 depicts photographs showing the results of evaluating the drug-loading capacity of hydrogel beads using an ultrafiltration filter.

### Mode for Invention

Preferred embodiments of the present invention will be described in more detail with reference to the accompanying drawings, and the descriptions of already known technical features will be omitted or abridged and simplified for the sake of brevity of description.

It should be noted that references to "an" or "one" embodiment of the invention herein are not necessarily to the same embodiment, and they mean "at least one."

In the following embodiments, terms such as "first" and "second" are not used in a limited sense, but are used for the purpose of distinguishing one component from another component.

In the embodiments below, singular expressions include plural expressions unless the context clearly indicates otherwise.

In the embodiments below, terms such as "include" and "have" are intended to denote the presence of characteristics or components described in the specification, but do not exclude the probability of addition of one or more other characteristics or components.

When some embodiments may be differently implemented, a particular process order may be performed differently from an order described. For example, two processes described in succession may be performed substantially simultaneously, or may be performed in an order opposite to an order described. That is, each step of the method described herein may be appropriately performed in any order, unless otherwise indicated herein or clearly contradicted by context.

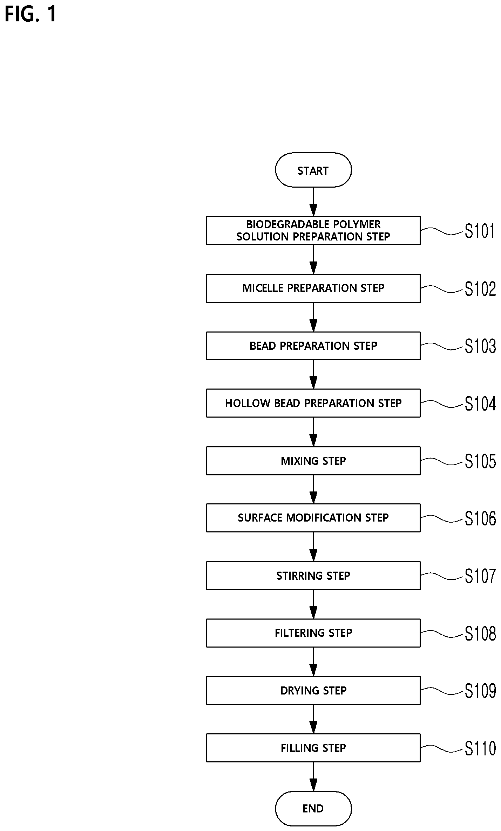

A method for producing a drug delivery system according to one embodiment of the present invention will be described with reference to the flow chart illustrated in FIG. 1 and with reference to the other drawings, and, for convenience, the method will be described in order.

### 1. Biodegradable polymer solution preparation step <S101>

In this step, a biodegradable polymer solution may be prepared by mixing a biodegradable polymer with an alkaline solution. In one embodiment, the biodegradable polymer contained in the biodegradable polymer solution may be at least one of hyaluronic acid and a hyaluronic acid salt. For example, the hyaluronic acid salt is a salt of hyaluronic acid, and examples thereof include both inorganic salts such as sodium hyaluronate, potassium hyaluronate, calcium hyaluronate, magnesium hyaluronate, zinc hyaluronate, and cobalt hyaluronate, and organic salts such as tetrabutylammonium hyaluronate. According to the implementation, the hyaluronic acid salt may be a combination of two or more of the above-described types. In one embodiment, the weight average molecular weight of the biodegradable polymer may be, but is not limited to, 100,000 to 15,000,000 g/mol.

In one embodiment, the alkaline solution may be prepared as a sodium hydroxide solution or a potassium hydroxide solution, but other types of alkaline solutions capable of dissolving hyaluronic acid may also be used.

In one embodiment, the biodegradable polymer solution may be prepared by mixing sodium hyaluronate and a 0.5 to 1 N sodium hydroxide solution so that the concentration of sodium hyaluronate becomes 10 w/v% and stirring the mixture for 3 to 4 hours. In this case, when the stirring temperature is maintained at a high temperature (for example, 60°C), the stirring time may be shortened.

### 2. Micelle preparation step <S102>

In this step, polymer micelles may be prepared by mixing and stirring the biodegradable polymer solution and an oil. That is, in this step, the biodegradable polymer may be adsorbed onto spherical oil particles while surrounding the particles to form polymer micelles. In one embodiment, the oil may be provided as heavy mineral oil.

In one embodiment, the biodegradable polymer solution and the heavy mineral oil may be mixed at a volume ratio of 1:5. If the amount of the mineral oil relative to the biodegradable polymer solution during mixing is larger than the above range (for example, the volume ratio is 1:6), there is a concern that the viscosity of the mixture may become excessively high during the stirring of the biodegradable polymer solution and the heavy mineral oil. In contrast, if the amount of the mineral oil relative to the biodegradable polymer solution is smaller than the above range (for example, the volume ratio is 1:4), the biodegradable polymer may aggregate during stirring and the diameter of the beads may become excessively large, making it difficult to prepare nanometer-sized beads.

In addition, in this step, the mixture of the biodegradable polymer solution and the mineral oil may be stirred at high speed for 30 minutes to 15 hours using an impeller that rotates at 1,000 to 2,000 rpm. Through high-speed stirring using the impeller, the biodegradable polymer surrounds the spherical oil particles to form polymer micelles.

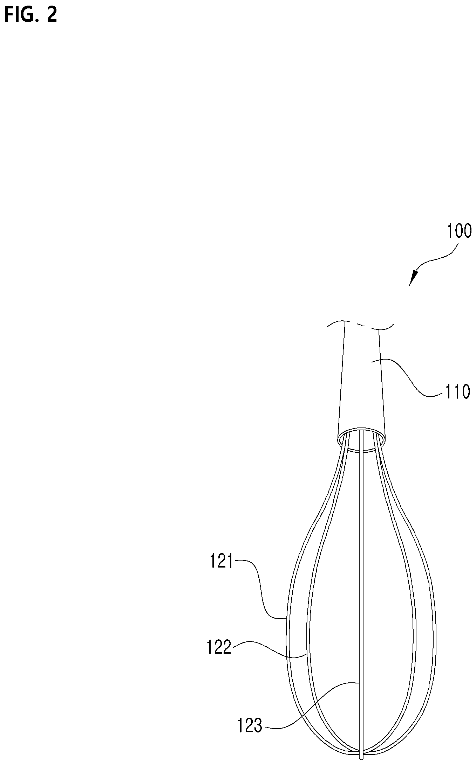

FIG. 2 is a perspective view schematically illustrating the structure of an impeller that is used in the micelle preparation step according to one embodiment of the present invention. Referring to FIG. 2, the impeller 100 may include: a rotational shaft 110 that rotates in one direction; and a plurality of wires 121, 122 and 123 that rotate together with the rotational shaft 110.

In one embodiment, two or three wires may be fixed to the rotational shaft 110 while intersecting each other at a predetermined angle. The central portion of the first wire 121 may be curved to form faced portions, and one end and the other end of the first wire 121 may be fixed to the rotational shaft 110. The first wire 121 has a structure in which the width increases downward from one end thereof, making it easy to stir the lower layer of the solution. The structure of the second wire 122 or the third wire 123 may also be fonned in the same manner as the first wire 121.

If the number of wires constituting the impeller 100 is smaller than 2, a problem arises in that the dispersion during stirring is reduced, making it difficult to form fine-sized polymer micelles, and if the number of wires is 4 or more, a problem arises in that aggregation occurs during stirring, forming lumps, and the lumps stick to the gaps between adjacent wires, making it difficult to form fine-sized polymer micelles. Thus, it is preferred to adjust the number of wires within the above-mentioned range.

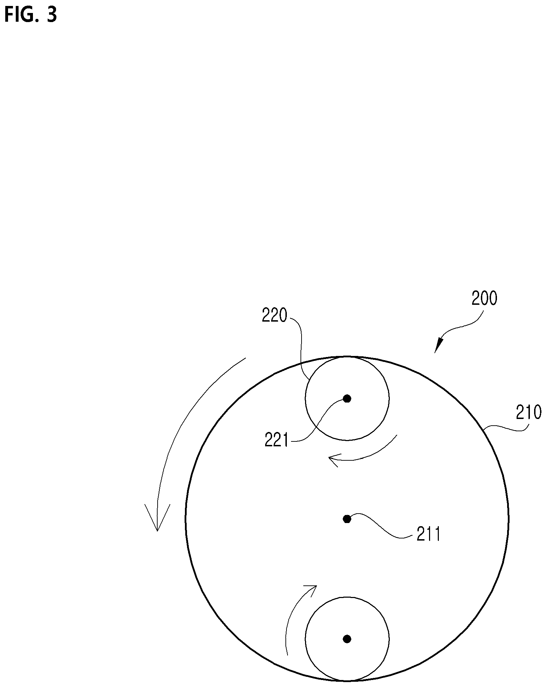

Meanwhile, in another embodiment, the mixture of the biodegradable polymer solution and the oil may be introduced into a ball mill container 220 of a ball mill device 200 and stirred, thereby preparing polymer micelles. As illustrated in FIG. 3, in one specific embodiment, the ball mill device 200 may include: a disk 210 that rotates in one direction; and a ball mill container 220 that is placed on the disk 210 and rotates in the other direction. For example, when the disk 210 rotates counterclockwise around the disk rotational shaft 211, the ball mill container 220 rotates clockwise with respect to the container rotational shaft 221. In addition, a plurality of balls may be provided inside the ball mill container 220. When the ball mill device 200 is used, the rotational speed of the disk 210 may be 500 rpm, the rotational speed of the ball mill container 220 may be 200 rpm, and the disk 210 and the ball mill container 220 may be rotated for 1 to 2 hours to stir the mixture of the biodegradable polymer solution and the mineral oil.

### 3. Bead preparation step <S103>

In this step, hydrogel beads filled with the oiltherein may be prepared by crosslinking the polymer micelles prepared in step S102. That is, in this step, the polymer micelles may be crosslinked by adding a crosslinking agent to the material stirred in step S102 and stirring the mixture at a predetermined speed (e.g., 1,000 to 2,000 rpm). In addition, this step may be performed at room temperature for 24 to 72 hours.

In one embodiment, the volume ratio between the mixture of the biodegradable polymer solution and the mineral oil and the crosslinking agent may be 100:2. If the amount of the mixture relative to the crosslinking agent during the crosslinking reaction is higher than the above range (for example, the volume ratio is 100:1), there is a concern that it is difficult to make the hydrogel beads having a spherical shape, and the impact resistance of the beads reduced, and thus the bead will easily break even with a slight impact. If the amount of the mixture relative to the crosslinking agent is smaller than the above range (for example, the volume ratio is 100:3), the color of the hydrogel beads may change to yellow, or the fluidity of the beads may decrease so that the volume expansion of the beads during hydration may be limited, and the crosslinking agent remaining in the beads may cause side effects *in vivo.* For this reason, it is preferable that the volume ratio between the mixture and the crosslinking agent be 100:2.

In one embodiment, the crosslinking agent may be at least one selected from the group consisting of 1-ethyl-3-(3-dimethylaminopropyl)carbodiimide hydrochloride, N,N'-dicyclohexylcarbodiimide, butanediol diglycidyl ether, polyethylene glycol diglycidyl ether, N-hydroxysuccinimide, N-hydroxysulfosuccinimide, and combinations thereof, but is not limited to this and it is also possible to use other types of known crosslinking agents.

### 4. Hollow bead preparation step <S104>

In this step, hollow beads may be prepared by removing the oil contained in the hydrogel beads crosslinked in step S103. In this case, the hollow bead refers to a bead from which the oil contained within the bead has been removed. In other words, the hollow bead is in a form in which the biodegradable polymer is formed like a ring only on the outside of the bead, and thus a space that may contain an additive may be formed in the bead.

In this step, when the oil contained in the bead is removed while washing the bead, water may be present in the bead, but in the filling step to be described below, the inside of the bead may be filled with an additive as the additive flows into the internal space of the bead due to the concentration gradient.

According to one embodiment, in this step, the hydrogel beads may be washed three times or more with purified water to remove the oil contained in the hydrogel beads, and the hydrogel beads that have been washed with purified water may be immersed and kept in ethanol for 30 minutes. By repeating the washing process using purified water and ethanol three times, the oil contained in the hydrogel beads, and the crosslinking agent, alkaline salt, unreacted crosslinking by-products, and impurities remaining in the hydrogel beads may be removed together.

### 5. Mixing step <S105>

In this step, the hollow beads prepared in step S104 may be mixed with a solvent (e.g., saline, phosphate buffered saline, etc.), thereby preparing a mixture. In one embodiment, the hollow beads and phosphate buffered saline may be mixed together so that the concentration of the hollow beads becomes 0.1 w/v%, followed by stirring for 3 to 4 hours, thereby preparing a mixture.

### 6. Surface modification step <S106>

In this step, the surface of the beads may be cationically modified by adding a cationic crosslinker and an activator to the mixture prepared in step S105. In one embodiment, the cationic crosslinker may be added so that the volume ratio between the mixture and the cationic crosslinker is 100: 1, and the activator may be added so that the volume ratio between the mixture and the activator is 100:0.2 to 100:0.4.

In one embodiment, the cationic crosslinker may be, but is not limited to, at least one selected from among lysine (e.g., L-lysine, poly-L-lysine, etc.), tris(2-ethylhexyl)amine, bis(2-ethylhexyl)amine, tris(2-pyridylmethyl)amine, poly(ethylene glycol)methyl ether amine, tris(2-aminoethyl)amine, hydrazine, poly(ethylene glycol) bis(amine), tris[2-(isopropylamino)ethyl]amine, and ethylenediamine.

Additionally, in one embodiment, the activator may be at least one selected from among 1-ethyl-3-(3-dimethylaminopropyl)carbodiimide hydrochloride, N,N'-dicyclohexylcarbodiimide, N,N'-carbonyldiimidazole, and N-hydroxysuccinimide, but other types of activators known in the art may also be used.

### 7. Stirring step <S107>

In this step, the mixture to which the cationic crosslinker and activator have been added may be stirred. For example, in this step, the mixture may be stirred at 100 to 200 rpm at room temperature (e.g., 25°C) for 12 to 24 hours.

### 8. Filtering step <S108>

In this step, the mixture stirred in step S107 may be filtered to remove the residual material contained in the mixture. In one embodiment, the residual material s may be filtered out using a semi-permeable membrane.

### 9. Drying step <S109>

In this step, the beads from which the residual material have been removed in step S 108 can be recovered and freeze-dried. For example, in this step, the beads may be frozen at a temperature range of - 80°C to -10°C, and dried at a temperature of 40°C to 20°C for 1 hour under a pressure of 10 mTorr or less.

### 10. Additive filling step <S110>

In this step, the inside of the hollow beads may be filled with an additive. This step may be performed after S109, or, according to the implementation, this step may be performed directly after step S104 while omitting steps S105 to S109.

In one embodiment, the additive may be at least one selected from among a drug, a protein, and a genetic material. For example, the drug may be as an anticancer drug. The anticancer drug may be selected from among cyclophosphamide, methotrexate, 5-fluorouracil, vinorelbine, doxorubicin, docetaxel, bleomycin, vinblastine, dacarbazine, mustine, vincristine, procarbazine, prednisolone, etoposide, cisplatin, epirubicin, capecitabine, folinic acid, oxaliplatin, gemcitabine, ifosfamide, etoposide, pembrolizumab, nivolumab, avelumab, durvalumab, atezolizumab, or combinations thereof, but is not limited only to the above-described examples.

In one embodiment, the genetic material may be at least one selected from among messenger RNA (mRNA) and plasmid DNA (pDNA).

According to one embodiment, in this step, when the additive solution and the bead solution are mixed together at a volume ratio of 1:1 and stirred for 2 to 3 hours, the inside of the hydrogel beads may be filled with the additive as the additive can penetrate into the hydrogel beads. In this case, the additive solution is a mixture of the additive and a first solution (e.g., phosphate buffered saline, borate buffer, saline, water for injection, etc.), and the bead solution is a mixture of the hollow beads and a second solution (e.g., phosphate buffered saline, borate buffer, saline, water for injection, or the like).

### 11. Sorting step <S111>

In this step, the hydrogel beads filled with the additive may be sorted by size using a sieve or porous membrane having pores of a predetermined size.

Hereinafter, the present invention will be described in more detail by way of specific examples. The following examples are merely examples to help understand the present invention, and the scope of the present invention is not limited or restricted thereto.

### Preparation of hydrogel beads containing specific substance

### <Example 1 and Comparative Examples 1 to 4>

A biodegradable polymer solution was prepared by mixing sodium hyaluronate and 1N sodium hydroxide solution so that the concentration of sodium hyaluronate was 10 w/v% and stirring the mixture for 3 to 4 hours. The prepared biodegradable polymer solution was mixed with heavy mineral oil, and the mixture of the biodegradable polymer solution and the mineral oil was stirred at high speed for 30 minutes to 15 hours using an impeller that rotated at 1,000 to 2,000 rpm. The volume ratio between the biodegradable polymer solution and the mineral oil upon mixing was set as shown in Table 1 below. The structure of the impeller used for stirring was the same as that in FIG. 2, and an impeller with three wires intersecting each other at a predetermined angle and fixed to a rotational shaft was used.

Thereafter, a crosslinking agent (butanediol diglycidyl ether) was added to the stirred mixture which was then stirred at 1000 rpm, and the polymer micelles were crosslinked at room temperature for 24 hours. In this case, the volume ratio between the mixture of the biodegradable polymer solution and the mineral oil and the crosslinking agent was set to 100:2. After completion of the crosslinking reaction, the prepared hydrogel beads were washed three times with purified water, and the hydrogel beads washed with purified water were immersed and kept in ethanol for 30 minutes. In this case, washing with purified water and ethanol was repeated three times.

Thereafter, the washed hydrogel beads were mixed with phosphate buffered saline so that the concentration of the beads was 0.1 w/v%, and then the mixture was stirred for 3 to 4 hours. Then, hydrazine, 1-ethyl-3-(3-dimethylaminopropyl)carbodiimide hydrochloride (EDC), N,N'-carbonyldiimidazole (CDI), and N-hydroxysuccinimide (NHS) were added to the mixture, followed by stirring at 100 rpm at room temperature for 12 hours. In this case, the amount of hydrazine added was set so that the volume ratio between the mixture of the hydrogel beads and phosphate buffered saline and hydrazine was 100: 1, and the amount of EDC added was set so that the volume ratio between the mixture of the hydrogel beads and phosphate buffered saline and EDC was 100:0.2. In addition, the amount of CDI added was set so that the volume ratio between the mixture of the hydrogel beads and phosphate buffered saline and CDI was 100:0.2, and the amount of NHS added was set so that the volume ratio between the mixture of the hydrogel beads and phosphate buffered saline and NHS was 100:0.4.

Thereafter, foreign substances were removed using a semi-permeable membrane, and the beads from which foreign substances had been removed were recovered, frozen at a temperature ranging from -80°C to -10°C, and dried at a temperature of -40°C to 20°C for 1 hour under a pressure of 10 mTorr or less. The freeze-dried beads were mixed with phosphate buffered saline so that the concentration of the beads was 2 mg/ml, thereby preparing a bead solution, and doxorubicin hydrochloride and phosphate buffered saline were mixed together so that the concentration of doxorubicin hydrochloride was 2 mg/ml, thereby preparing an additive solution. Then, the additive solution and the bead solution were mixed together at a volume ratio of 1:1 and stirred for 2 to 3 hours. Thereafter, the resulting hydrogel beads filled with doxorubicin hydrochloride were sorted using a sieve with a size of 30 to 40 µm.

**[Table 1]**

| | Example 1 | Comparative Example 1 | Comparative Example 2 | Comparative Example 3 | Comparative Example 4 |
|---|---|---|---|---|---|
| Biodegradable polymer solution: mineral oil | 1:5 | 1:3 | 1:4 | 1:6 | 1:7 |

### <Example 2 and Comparative Examples 5 to 8>

Hydrogel beads were prepared in the same manner as in Example 1, except that the volume ratio of the crosslinking agent was set as described in Table 2 below.

**[Table 2]**

| Volume ratio between mixture (mixture of biodegradable polymer solution and mineral oil) and crosslinking agent | | | | |
|---|---|---|---|---|
| Example 2 | Comparative Example 5 | Comparative Example 6 | Comparative Example 7 | Comparative Example 8 |
| 100:2 | 100:1 | 100:0.5 | 100:2.5 | 100:3 |

### Evaluation of properties of hydrogel beads depending on amount of mineral oil added

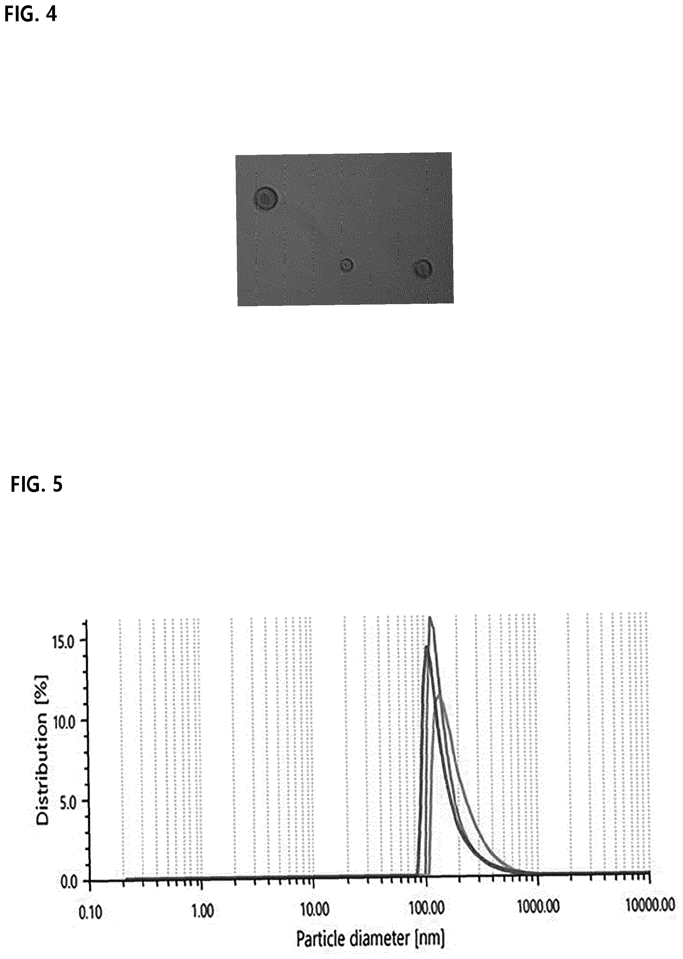

FIG. 4 is a confocal micrograph of the hydrogel beads according to Example 1 of the present invention, taken at ×100 magnification. As a result of observing the hydrogel beads according to Example 1 using a confocal microscope, it can be seen that hydrogel beads having a round and smooth spherical shape like balls were formed. In comparison, it was confirmed that, in the case of Comparative Examples 1 and 2, in which the amount of the mineral oil relative to the amount of the biodegradable polymer solution was relatively small, aggregation and clumping of the biodegradable polymer occurred during stirring of the mineral oil and biodegradable polymer solution, and a large number of hydrogel beads with a diameter greater than 500 µm were formed. In addition, it was confirmed that, in the case of Comparative Examples 3 and 4, as the amount of the mineral oil relative to the amount of the biodegradable polymer solution became relatively large, the dispersion during stirring decreased and a large number of beads stuck to each other were formed.

### Evaluation of properties of hydrogel beads depending on content of crosslinking agent

In order to evaluate the properties of hydrogel beads depending on the content of the crosslinking agent, the following experiment was conducted. The hydrogel beads of each of the Examples and the Comparative Example were observed with a confocal microscope (×100) to check the shape of the beads. If the bead shape was determined to be spherical, the shape was evaluated as good, and if the bead shape was distorted or uneven, the shape was evaluated as poor. In addition, it was checked whether discoloration occurred during the stirring process after adding the crosslinking agent to the stirred mixture. If no discoloration occurred, it was evaluated as good. In contrast, if discoloration occurred, it was evaluated as poor. In addition, the hydrogel beads of each of the Examples and the Comparative Examples were hydrated by immersion for 24 hours in phosphate buffered saline that had been left in a 37°C constant-temperature water bath for 2 hours, and then the area of the hydrogel bead was measured using a microscopic infrared spectrometer. If the area (µm²) of the hydrogel bead after hydration increased by 20 times or more compared to the area of the hydrogel bead before hydration, the expansion of the bead was evaluated as good. In contrast, if the area of the bead increased by less than 20 times, the expansion of the bead was evaluated as poor. The results for each test are shown in Table 3 below.

**[Table 3]**

| | Example 2 | Comparative Example 5 | Comparative Example 6 | Comparative Example 7 | Comparative Example 8 |
|---|---|---|---|---|---|
| Bead shape | Good | Poor | Poor | Good | Good |
| Whether discoloration occurred | Good | Good | Good | Poor | Poor |
| Expansion | Good | Good | Good | Poor | Poor |

As shown in Table 3 above, it was confirmed that, in the case of Example 2, the bead shape was good, no discoloration occurred, and the expansion of the bead was good, whereas, in the case of Comparative Example 5, the bead had a distorted appearance, and in the case of Comparative Example 6, the bead had an uneven or distorted surface, indicating that the surface did not have a round shape like balls. It was confirmed that, in the case of Comparative Example 7, the color changed to light yellow during the stirring process after adding the crosslinking agent to the stirred mixture, and in the case of Comparative Example 8, the color also changed to light yellow. In addition, it was confirmed that, in the case of Comparative Examples 7 and 8, as the amount of crosslinking agent added increased, the degree of crosslinking of the beads increased and the expansion of the beads decreased.

### Analysis of diameter of hydrogel beads

The diameter of the hydrogel beads according to Example 1 was analyzed using a nano-particle size analyzer. FIG. 5 is a graph showing the results of analyzing the hydrogel beads according to Example 1 of the present invention using a nano-particle size analyzer. As shown in FIG. 5, it can be seen that the hydrogel beads according to Example 1 have particles having a particle size of 100 to 300 nm, which account for 95% of the total particles.

### Evaluation of drug-loading capacity of hydrogel beads

### <Example 3>

A biodegradable polymer solution was prepared by mixing sodium hyaluronate and a 1N sodium hydroxide solution so that the concentration of sodium hyaluronate was 10 w/v% and stirring the mixture for 3 to 4 hours. The prepared biodegradable polymer solution was mixed with heavy mineral oil, and the mixture of the biodegradable polymer solution and the mineral oil was stirred at high speed for 30 minutes to 15 hours using an impeller that rotated at 1,000 to 2,000 rpm. The volume ratio between the biodegradable polymer solution and mineral oil was set to 1:5. The structure of the impeller used for stirring was the same as that in FIG. 2, and the impeller with three wires intersecting each other at a predetermined angle and fixed to a rotational shaft was used.

Thereafter, a crosslinking agent (butanediol diglycidyl ether) was added to the stirred mixture which was then stirred at 1,000 rpm, and the polymer micelles were crosslinked at room temperature for 24 hours. In this case, the volume ratio between the mixture of the biodegradable polymer solution and the mineral oil and the crosslinking agent was set to 100:2. After completion of the crosslinking reaction, the prepared hydrogel beads were washed three times with purified water, and the hydrogel beads washed with purified water were immersed and kept in ethanol for 30 minutes. In this case, washing with purified water and ethanol was repeated three times.

Thereafter, the washed hydrogel beads were mixed with phosphate buffered saline so that the concentration of the beads was 0.1 w/v%, and then the mixture was stirred for 3 to 4 hours. Then, hydrazine, 1-ethyl-3-(3-dimethylaminopropyl)carbodiimide hydrochloride (EDC), N,N'-carbonyldiimidazole (CDI), and N-hydroxysuccinimide (NHS) were added to the mixture, followed by stirring at 100 rpm at room temperature for 12 hours. In this case, the amount of hydrazine added was set so that the volume ratio between the mixture of the hydrogel beads and phosphate buffered saline and hydrazine was 100: 1, and the amount of EDC added was set so that the volume ratio between the mixture of the hydrogel beads and phosphate buffered saline and EDC was 100:0.2. In addition, the amount of CDI added was set so that the volume ratio between the mixture of the hydrogel beads and phosphate buffered saline and CDI was 100:0.2, and the amount of NHS added was set so that the volume ratio between the mixture of the hydrogel beads and phosphate buffered saline and NHS was 100:0.4.

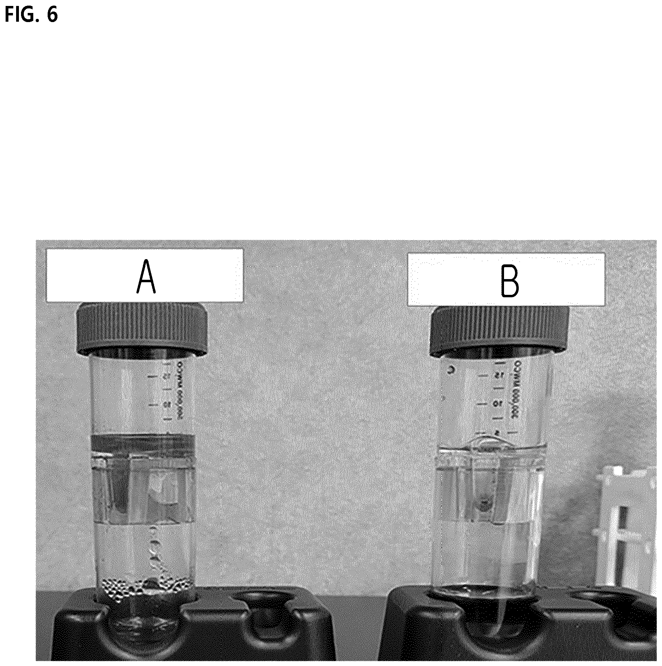

Thereafter, foreign substances were removed using a semi-permeable membrane, and the beads from which foreign substances have been removed were recovered, fiozen at a temperature ranging from -80°C to -10°C, and dried at a temperature of -40°C to 20°C for 1 hour under a pressure of 10 mTorr or less. 100 mg of the freeze-dried beads were mixed with a doxorubicin hydrochloride solution (the doxorubicin hydrochloride solution was prepared by mixing 10 mg of doxorubicin hydrochloride with 10 ml of phosphate buffered saline), and the mixture was placed in a Sartorius ultrafiltration filter (300,000 Da) and centrifuged at 1,000 rpm for 3 minutes, and then the filtered state was checked. FIG. 6 depicts photographs showing the results of evaluating the drug-loading capacity of the hydrogel beads using the ultrafiltration filter. FIG. 6A is a photograph showing the results of filtering the mixture of the hydrogel beads and the doxorubicin hydrochloride solution through the ultrafiltration filter. As a result of comparing the values before and after filtering, it was confirmed that 70% or more of the total volume did not pass through the ultrafiltration membrane. Therefore, it can be seen that the hydrogel beads according to Example 3 have a drug-loading capacity of 7 mg or more per 100 mg of the hydrogel beads.

### <Comparative Example 9>

A doxorubicin hydrochloride solution was prepared by mixing 10 mg of doxorubicin hydrochloride with 10 ml of phosphate buffered saline, placed in a Sartorius ultrafiltration filter (300,000 Da), and centrifuged at 1,000 rpm for 3 minutes, and then the filtered state was checked. FIG. 6B is a photograph showing the results of filtering the doxorubicin hydrochloride solution according to Comparative Example 9 through the ultrafiltration filter. As a result of comparing the values before and after filtering, it was confirmed that most of the solution passed through the ultrafiltration filter.

As described above, according to various embodiments of the present invention, it is possible to achieve a sustained-release function of sustainedly releasing a functional additive from beads over time by making biodegradable hollow beads and filling the inside of the biodegradable hollow beads with the functional additive.

In addition, according to various embodiments of the present invention, it is possible to produce a large amount of hydrogel beads having a round surface and a smooth spherical shape like balls. In addition, since the surface of the hydrogel beads has a predetermined strength as a result of crosslinking, the beads may retain their shape for a long time without being easily deformed *in vivo.*

It is structurally difficult for conventional beads, obtained by forming a hydrogel and then making the hydrogel into a bead shape through mechanical processing, to be loaded with a useful substance or exhibit a sustained-release function because a crosslinked hydrogel consisting of the same component as the surface is contained in the beads. However, according to various embodiments of the present invention, it is possible to achieve a sustained-release function of sustainedly releasing various types of functional additives from beads over time by making hollow beads and filling the inside of the hollow beads with the functional additives.

In addition, according to various embodiments of the present invention, side effects caused by the crosslinking agent may be prevented by controlling the content of the crosslinking agent. Furthermore, compared to conventional beads obtained by forming a hydrogel and then making the hydrogel into a bead shape through mechanical processing, even when using the same content of a crosslinking agent, the hydrogel beads according to various embodiments of the present invention have a crosslinking reaction that occurs intensively on the surface of the bead, so that the crosslinking degree of the surface layer is increased, and thus the beads may retain their shape in the human body for a longer period of time than the conventional beads in which a crosslinking reaction has occurred in the inside and outside of the bead.

Although specific embodiments of the present invention have been described above in detail with reference to the accompanying drawings, the above-described embodiments are only preferred examples of the present invention. Therefore, it should be understood that the present invention is not limited only to the above-described embodiments, and the scope of the present invention should be defined by the appended claims and equivalents thereto.

## Claims

1. A method for producing a drug delivery system, the method comprising:
a micelle preparation step of preparing polymer micelles by mixing and stirring a biodegradable polymer solution and an oil;
a bead preparation step of preparing hydrogel beads filled with the oil therein by crosslinking the polymer micelles;
a hollow bead preparation step of preparing hollow beads by removing the oil contained in the hydrogel beads; and
a filling step of filling the hollow beads with an additive.

2. The method of claim 1, wherein a crosslinking agent that is used in the bead preparation step is at least one selected from the group consisting of 1-ethyl-3-(3-dimethylaminopropyl)carbodiimide hydrochloride, N,N'-dicyclohexylcarbodiimide, butanediol diglycidyl ether, polyethylene glycol diglycidyl ether, N-hydroxysuccinimide, N-hydroxysulfosuccinimide, and combinations thereof.

3. The method of claim 1, wherein the biodegradable polymer contained in the biodegradable polymer solution is at least one selected from among hyaluronic acid and a hyaluronic acid salt.

4. The method of claim 1, wherein the additive is at least one selected from among a drug, a protein, and a genetic material.

5. The method of claim 4, wherein the drug is an anticancer drug.

6. The method of claim 4, wherein the genetic material is at least one selected from among messenger RNA (mRNA) and plasmid DNA (pDNA).

7. The method of claim 1, further comprising:
a mixing step of preparing a mixture by mixing the hollow beads with a solvent;
a surface modification step of adding a cationic crosslinker and an activator to the mixture;
a stirring step of stirring the mixture;
a filtering step of filtering the mixture to remove residual material; and
a drying step for freeze-drying the filtered beads.

8. The method of claim 7, wherein the cationic crosslinker is at least one selected from among lysine, tris(2-ethylhexyl)amine, bis(2-ethylhexyl)amine, tris(2-pyridylmethyl)amine, poly(ethylene glycol)methyl ether amine, tris(2-aminoethyl)amine, hydrazine, poly(ethylene glycol)bis(amine), tris[2-(isopropylamino)ethyl]amine, and ethylenediamine.

9. The method of claim 7, wherein the activator is at least one selected from among 1-ethyl-3-(3-dimethylaminopropyl)carbodiimide hydrochloride, N,N'-dicyclohexylcarbodiimide, N,N'-carbonyldiimidazole, and N-hydroxysuccinimide.

10. A drug delivery system produced by the method of any one of claims 1 to 9.
